# EUROPEAN PATENT APPLICATION

(11) **EP 4 610 574 A1**
(43) Date of publication of application: **03.09.2025**
(21) Application number: 23881376.0
(22) Date of filing: 04.08.2023
(51) Int. Cl.: F24F 8/22, F24F 11/89, F24F 13/30

(54) **PULSE LAMP BODY, PULSE STERILIZATION MODULE AND AIR CONDITIONER**

(30) Priority: 27.10.2022 CN 202211329568; 27.10.2022 CN 202222875828 U
(71) Applicant: Midea Group Wuhan Refrigeration Equipment Co., Ltd., Wuhan City, Hubei 430056 (CN); GD Midea Air-Conditioning Equipment Co., Ltd., Foshan, Guangdong 528311 (CN)
(72) Inventor: HU, Haitao, Wuhan, Hubei 430056 (CN); TAN, Chaohua, Wuhan, Hubei 430056 (CN); YANG, Ganggang, Wuhan, Hubei 430056 (CN); MAO, Xianyou, Wuhan, Hubei 430056 (CN); SHEN, Zhicong, Wuhan, Hubei 430056 (CN)
(74) Representative: Ran, Handong
(86) International application number: PCT/CN2023/111340
(87) International publication number: WO 2024/087790

(57) **Abstract**

A pulse lamp body (24), a pulse sterilization module (20) and an air conditioner. The pulse lamp body (24) comprises: a lamp tube (24a), a light-emitting body (24b) and a sealing joint (25); one end of the lamp tube (24a) is open, and the other end of the lamp tube (24a) is closed; the light-emitting body (24b) is provided in the lamp tube (24a) and is connected to an input wire harness (22); the sealing joint (25) is provided at the open end of the lamp tube (24a), and the input wire harness (22) is led out through the sealing joint (25).

## Description

### CROSS-REFERENCE TO RELATED APPLICATION

This application is based on and claims priority from Chinese Patent Applications No. 202211329568.9, and No. 20222875828.4, both filed on October 27, 2022, the entire contents of which are incorporated herein by reference.

### FIELD

The present application relates to the field of sterilization technologies, and more particularly, to a pulse lamp, a pulse sterilization module, and an air conditioner.

### BACKGROUND

In the related art, a pulse lamp is generally composed of a lamp tube, a light-emitting body, a wire harness, and end covers. The end covers are located at two ends of the lamp tube. Usually, the lamp tube has two open ends for leading out the wire harness, and the end covers are arranged at the two open ends of the lamp tube, allowing an assembly efficiency of the pulse lamp to be low. In addition, after long-term use of the lamp tube with two open ends, it is difficult to ensure a sealing performance of the lamp tube, and condensed water is likely to be generated inside the lamp tube in an alternating cold and hot environment, which deteriorates a light transmission effect of the lamp tube and make the light-emitting body prone to moisture, affecting a service life of the pulse lamp and failing to ensure a safety of using the pulse lamp.

### SUMMARY

The present application aims at solving at least one of the technical problems existing in the related art. To this end, an object of the present application is to provide a pulse lamp. Since the lamp tube has an open end and a closed end, and a sealing connector is disposed at the open end of the lamp tube, an assembly efficiency of the pulse lamp is high, and the sealing connector can seal the open end of the lamp tube to seal an inner space of the lamp tube. On the one hand, condensed water generated in the lamp tube can be prevented from affecting light transmission of the lamp tube, and on the other hand, a sealing performance and a moisture-proof performance of the lamp tube can be enhanced, thereby alleviating oxidation of an input wire harness. In addition, a service life of a light-emitting body and the input wire harness can be ensured, thus ensuring a service life of the pulse lamp and a safety of the pulse lamp.

The present application further provides a pulse sterilization module having the above-described pulse lamp.

The present application further provides an air conditioner having the above-described pulse sterilization module.

According to the pulse lamp of embodiments in a first aspect of the present application, the pulse lamp includes: a lamp tube having an open end and a closed end; a light-emitting body disposed in the lamp tube, the light-emitting body being connected with an input wire harness; a sealing connector disposed at the open end of the lamp tube, the input wire harness being led out through the sealing connector.

According to the pulse lamp of the embodiments of the present application, since the lamp tube has the open end and the closed end, and the sealing connector is disposed at the open end of the lamp tube, so that the assembly efficiency of the pulse lamp is high. In addition, the sealing connector can seal the open end of the lamp tube to seal the inner space of the lamp tube. On the one hand, condensed water generated in the lamp tube can be prevented from affecting the light transmission of the lamp tube, and on the other hand, the sealing performance and the moisture-proof performance of the lamp tube can be enhanced, thereby alleviating the oxidation of the input wire harness. In addition, the service life of the light-emitting body and the input wire harness can be ensured, thus ensuring the service life of the pulse lamp and the safety of the pulse lamp.

According to some embodiments of the present application, the input wire harness includes a positive input wire harness, a negative input wire harness, and a signal input wire harness. The positive input wire harness and the negative input wire harness are respectively connected to two opposite sides of the light-emitting body. A connection between the negative input wire harness and the light-emitting body is located at a side of the light-emitting body adjacent to the closed end of the lamp tube. A wire routing space is defined between the lamp tube and the light-emitting body. The negative input wire harness is routed into the sealing connector through the wire routing space.

According to some optional embodiments of the present application, a part of the negative input wire harness located in the wire routing space is a first wire harness segment. At least part of the first wire harness segment facing towards the light-emitting body in a radial direction of the lamp tube is sleeved with an insulating sleeve.

In some optional embodiments of the present application, a part of the negative input wire harness located in the sealing connector is a second wire harness segment. The insulating sleeve extends into the sealing connector and is sleeved on an outer peripheral side of the second wire harness segment.

According to some embodiments of the present application, the sealing connector includes a sealing soft plug. The sealing soft plug includes a blocking portion received in the lamp tube and blocks the open end of the lamp tube. The sealing soft plug further has a first routing channel. At least part of the first routing channel is located in the blocking portion. A part of the input wire harness is routed along the first routing channel.

According to some optional embodiments of the present application, the sealing soft plug has a sealing engagement groove that is of an annular shape. The open end of the lamp tube is inserted into the sealing engagement groove. A part of the sealing soft plug located at an inner peripheral side of the sealing engagement groove is constructed as the blocking portion.

In some optional embodiments of the present application, an engagement length between the sealing soft plug and the lamp tube ranges from 12 mm to 20 mm.

In some optional embodiments of the present application, the first routing channel includes a first sub-routing channel, a second sub-routing channel, and a third sub-routing channel arranged at intervals. The input wire harness includes a positive input wire harness routed along the first sub-routing channel, a negative input wire harness routed along the second sub-routing channel, and a signal input wire harness routed along the third sub-routing channel.

In some optional embodiments of the present application, the sealing soft plug has a wire outlet groove formed at an axial end surface of the sealing soft plug away from the light-emitting body. The input wire harness is led out through the wire outlet groove. The sealing connector further includes a sealing adhesive filled in the wire outlet groove to at least seal a gap between the input wire harness and an inner wall of the wire outlet groove. An adhesive injection port is defined between the input wire harness and an inner peripheral wall of the wire outlet groove. The adhesive injection port is configured for adhesive injection to form the sealing adhesive.

According to some optional embodiments of the present application, the pulse lamp further includes a protective rubber sleeve sleeved on the closed end of the lamp tube.

According to the pulse sterilization module of embodiments in a second aspect of the present application, the pulse sterilization module includes: a lamp cover; the pulse lamp according to the above-described embodiments in the first aspect of the present application, the pulse lamp being disposed in the lamp cover; and a lamp support, the pulse lamp being fixedly mounted in the lamp cover through the lamp support.

According to the pulse sterilization module of the embodiments of the present application, by providing the above-described pulse lamp in the pulse sterilization module, since the lamp tube has the open end and the closed end, and the sealing connector is disposed at the open end of the lamp tube, so that the assembly efficiency of the pulse lamp is high. In addition, the sealing connector can seal the open end of the lamp tube to seal the inner space of the lamp tube. On the one hand, condensed water generated in the lamp tube can be prevented from affecting the light transmission of the lamp tube, and on the other hand, the sealing performance and the moisture-proof performance of the lamp tube can be enhanced, thereby alleviating the oxidation of the input wire harness. In addition, the service life of the light-emitting body and the input wire harness can be ensured, thus ensuring the service life of the pulse lamp and the safety of the pulse lamp.

According to some embodiments of the present application, the lamp support is provided with a wire routing buckle at an end of the lamp support adj acent to the sealing connector. A part of the input wire harness located outside the pulse lamp is routed through the wire routing buckle.

According to some embodiments of the present application, the lamp support has a lamp body groove and is provided with positioning clips. The positioning clips are located at two opposite sides of the lamp body groove in a length direction of the lamp body groove. Two ends of the lamp tube are positioned by the positioning clips.

According to the air conditioner of embodiments in a third aspect of the present application, the air conditioner includes: a housing having an air inlet and an air outlet; a heat exchanger component and an air duct component that are arranged in the housing; the pulse sterilization module according to the above-described embodiments in the second aspect of the present application, the pulse sterilization module being disposed in the housing and adapted to emit pulsed light into the housing.

According to the air conditioner of the embodiments of the present application, by providing the above-described pulse sterilization module in the air conditioner, since the lamp tube has the open end and the closed end, and the sealing connector is disposed at the open end of the lamp tube, the assembly efficiency of the pulse lamp is high, and the sealing connector can seal the open end of the lamp tube to seal the inner space of the lamp tube. On the one hand, condensed water generated in the lamp tube can be prevented from affecting the light transmission of the lamp tube, and on the other hand, the sealing performance and the moisture-proof performance of the lamp tube can be enhanced, thereby alleviating the oxidation of the input wire harness. In addition, the service life of the light-emitting body and the input wire harness can be ensured, thus ensuring the service life of the pulse lamp and the safety of the pulse lamp.

According to some embodiments of the present application, an air duct cavity is defined between the heat exchanger component and the air duct component. The air duct component includes a fan, an air duct volute, and an air duct partition. The air duct partition is connected to a side of the air duct volute adjacent to the heat exchanger component and abuts against the heat exchanger component to divide the air duct cavity into two air duct sub-cavity. The fan has a wind wheel disposed in the air duct sub-cavities. The pulse sterilization module is disposed at an upper part of the air duct partition.

According to some optional embodiments of the present application, the air conditioner further includes a pulse electric control box electrically connected to the pulse sterilization module. The air duct volute is provided with a motor cover at an outer wall of the air duct volute. The pulse electric control box is fixedly mounted at the motor cover. The fan has a motor disposed in the motor cover and connected to the wind wheel.

Additional aspects and advantages of the present application will be given at least in part in the following description, or will become apparent at least in part from the following description, or can be learned from practicing of the present application.

### BRIEF DESCRIPTION OF THE DRAWINGS

The above and/or additional aspects and advantages of the present application will become apparent and more understandable from the following description of embodiments taken in conjunction with the accompanying drawings.
FIG. 1 is a perspective view of an indoor unit of an air conditioner according to some embodiments of the present application.
FIG. 2 is an exploded view of the indoor unit of the air conditioner in FIG. 1.
FIG. 3 is a partial structural view of the indoor unit of the air conditioner in FIG. 1, in which part of a housing is removed and an internal structure is shown.
FIG. 4 is an enlarged view of part A in FIG. 3.
FIG. 5 is a sectional view of an upper part of the indoor unit of the air conditioner in FIG. 1.
FIG. 6 is an enlarged view of part B in FIG. 5.
FIG. 7 is an assembly view of a pulse sterilization module and a pulse electric control box according to some embodiments of the present application.
FIG. 8 is a schematic view of a wire routing of a first connection wire harness in FIG. 7.
FIG. 9 is a schematic view of a wire routing of a second connection wire harness in FIG. 7.
FIG. 10 is a partial structural view of an air duct component in FIG. 8.
FIG. 11 is a schematic structural view of a pulse sterilization module and a pulse electric control box in FIG. 8.
FIG. 12 is a sectional view taken along line C-C in FIG. 11.
FIG. 13 is a sectional view taken along line E-E in FIG. 11.
FIG. 14 is a sectional view taken along line F-F in FIG. 11.
FIG. 15 is a schematic view of the pulse electric control box and pulse sterilization module in FIG. 11 from another angle.
FIG. 16 is a schematic structural view of the pulse electric control box in FIG. 15.
FIG. 17 is a schematic structural view of a lamp cover in FIG. 15.
FIG. 18 is a schematic view of an engagement between a pulse lamp and a lamp support according to some embodiments of the present application.
FIG. 19 is an exploded view of the pulse lamp and lamp support in FIG. 18.
FIG. 20 is a sectional view of the pulse lamp and lamp support in FIG. 18.
FIG. 21 is a schematic structural view of the lamp support in FIG. 19.
FIG. 22 is a sectional view of a protective rubber sleeve, a lamp tube, a sealing soft plug, and a sealing adhesive in FIG. 19.
FIG. 23 is a schematic view of the sealing soft plug in FIG. 19 from another angle.

### Reference numerals of the accompanying drawings:

100, indoor unit;
10, housing; 11, rear housing; 12, air inlet grille; 13, air inlet; 14, top cover; 15, air outlet frame; 151, frame; 152, louver blade; 153, air guide plate; 16, lower panel; 17, base; 18, upper panel; 19, air outlet;
20, pulse sterilization module; 21, lamp cover; 212, support portion; 2121, first wire routing groove; 2122, second positioning hole; 213, light outlet; 214, first clip; 215, positioning post; 216, third fixing hole; 217, fixing post; 2171, first fixing hole; 218, third wire routing buckle; 219, first limiting protrusion; 22, input wire harness ; 221, positive input wire harness; 222, negative input wire harness; 2221, first wire harness segment; 2222, second wire harness segment; 223, signal input wire harness; 224, insulating sleeve; 225, input wire protective sleeve; 23, lamp support; 230, wire routing buckle; 231, first positioning hole; 232, second fixing hole; 233, lamp body groove; 234, positioning clips; 235, positioning clip plate;
24, pulse lamp; 24a, lamp tube; 24b, light-emitting body; 241, open end; 242, closed end; 243, positive electrode; 244, negative electrode; 245, wire routing space; 25, sealing connector; 26, sealing soft plug; 260, first routing channel; 261, first sub-routing channel; 262, second sub-routing channel; 263, third sub-routing channel; 264, blocking portion; 265, sealing engagement groove; 266, wire outlet groove; 267, adhesive injection port; 26a, first lead hole; 26b, second lead hole; 26c, third lead hole; 27, sealing adhesive; 270, second routing channel; 271, fourth sub-routing channel; 272, fifth sub-routing channel; 273, sixth sub-routing channel; 28, protective rubber sleeve
30, pulse electric control box; 31, first connection wire harness; 32, second connection wire harness; 33, wire outlet hole; 34, mounting lug; 341, first mounting hole;
40, air duct component; 4, air duct volute; 41, second wire routing buckle; 43, fan; 430, wind wheel; 431, first wind wheel; 432, second wind wheel; 44, motor; 441, first motor; 442, second motor; 45, motor cover ; 46, front cover body; 47, rear cover body; 471, first mounting post; 4711, second mounting hole; 472, limiting buckle; 473, first limiting rib plate; 474, second limiting rib plate; 475, second wire routing groove; 48, air duct partition; 480, mounting notch; 481, mounting wall; 482, third routing groove; 483, first positioning protrusion; 484, first clip groove; 485, first limiting groove; 486, fourth fixing hole;
50, heat exchanger component; 51, heat exchanger; 52, heat exchanger support; 53, electric auxiliary heating component;
60, air duct cavity; 6, air duct sub-cavity; 61, first air duct sub-cavity; 62, second air duct sub-cavity;
71, a first fastener; 72, a second fastener; 73, a third fastener.

### DETAILED DESCRIPTION OF THE EMBODIMENTS

Embodiments of the present application will be described in detail below with reference to examples thereof as illustrated in the accompanying drawings, throughout which same or similar elements, or elements having same or similar functions, are denoted by same or similar reference numerals. The embodiments described below with reference to the drawings are illustrative only, and are intended to explain, rather than limiting, the present application.

A pulse lamp 24 according to the embodiments of the present application is described below with reference to the accompanying drawings.

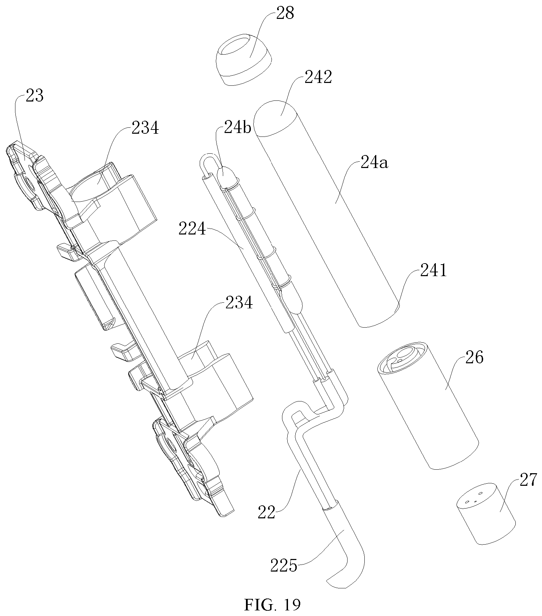

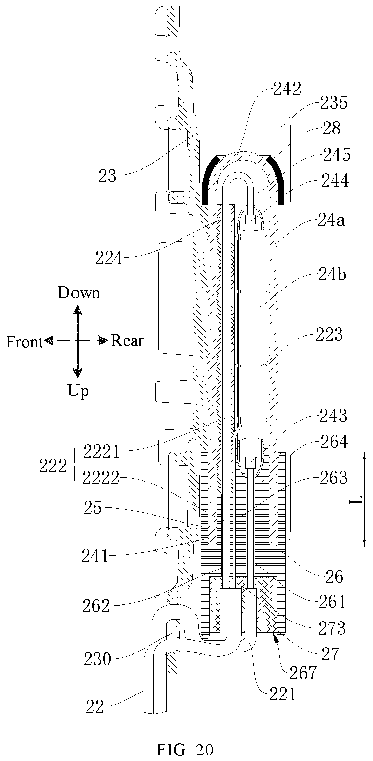

Referring to FIG. 19 and FIG. 20, the pulse lamp 24 according to the embodiments in a first aspect of the present application includes: a lamp tube 24a, a light-emitting body 24b, and a sealing connector 25. The lamp tube 24a has an open end and a closed end. The light-emitting body 24b is disposed in the lamp tube 24a and is connected with an input wire harness 22. For example, the light-emitting body 24b can extend into the lamp tube 24a from the open end 241 of the lamp tube 24a. Two opposite sides of the light-emitting body 24b are respectively connected with the input wire harnesses 22. By providing the light-emitting body 24b in the lamp tube 24a, the lamp tube 24a can be used to protect the light-emitting body 24b, prevent the light-emitting body 24b from being affected by external condensation water or dust, etc., to ensure a service life of the light-emitting body 24b and make the light-emitting body 24b insulated from objects (e.g. wire harness) outside the lamp tube 24a, thereby preventing heating, oxidation and other problems caused by direct contact between objects outside the lamp tube 24a and the light-emitting body 24b, so as to avoid potential safety hazards.

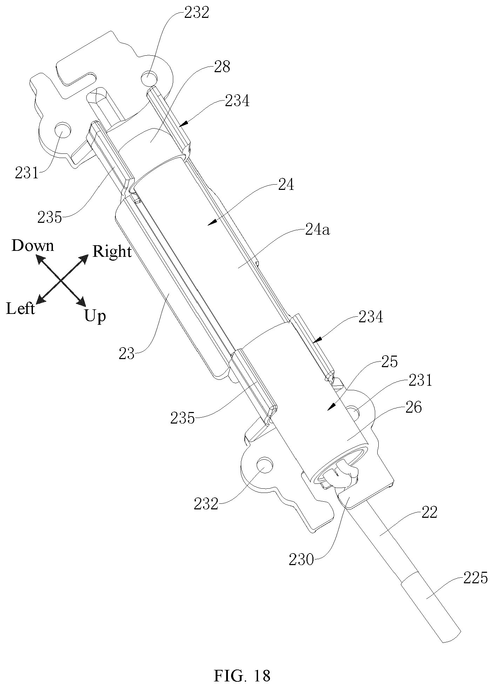

Referring to FIG. 18 to FIG. 20, the sealing connector 25 is disposed at the open end 241 of the lamp tube 24a. The input wire harness 22 is led out through the sealing connector 25. It is easier to wire with the input wire harness 22. Since an end of the lamp tube 24a is open, air can enter the lamp tube 24a from the open end 241 of the lamp tube 24a, condensed water is easily generated in the lamp tube 24a in an alternating cold and hot environment, which affects light transmission of the lamp tube 24a, allows the input wire harness 22 to easily oxidized, and affects the service life of the light-emitting body 24b. By providing the sealing connector 25 at the open end 241 of the lamp tube 24a, the light-emitting body 24b is prevented from coming out of the open end 241 of the lamp tube 24a, and the sealing connector 25 can seal the open end 241 of the lamp tube 24a. In this way, the lamp tube 24a and the light-emitting body 24b inside the lamp tube 24a can be prevented from being affected by condensed water.

In the related art, two ends of a lamp tube are open, and when assembling the pulse lamp, end covers need to cover two open ends of the lamp tube. In the present application, since the lamp tube 24a has the open end and the closed end, the sealing connector 25 is only required to be disposed at the open end 241 of the lamp tube 24a when assembling the pulse lamp 24, which reduces an assembly process compared with the related art, and allows the assembly efficiency of the pulse lamp 24 to be higher. In addition, the lamp tube 24a has the open end and the closed end, and compared with the lamp tube 24a having two open ends, the lamp tube 24a of the present application has better sealing performance. Since the open end 241 of the lamp tube 24a is sealed by the sealing connector 25, an insulating performance and a moisture-proof performance of the lamp tube 24a can be further enhanced.

The light-emitting body 24b of the above-described pulse lamp 24 can emit pulsed light. When the pulsed light is irradiated on a surface of microorganisms (such as bacteria and viruses), substances in the microorganisms can be changed and inactivated, allowing various microorganisms to be killed, thereby purifying and sterilizing parts irradiated by the pulsed light. Therefore, a sterilization effect is good and a sterilization efficiency is high. In addition, when the pulsed light is irradiated in air, some harmful gases (such as VOC gases) in the air can further be decomposed under irradiation of the pulsed light, thereby purifying the air. For example, the pulse lamp 24 can be used in the air outlet channel of an air conditioner, which can purify and sterilize the air outlet channel and further purify and sterilize the air in the air outlet channel, thereby enhancing a quality of the air blown into a room.

According to the pulse lamp 24 of the embodiments of the present application, since the lamp tube 24a has the open end and the closed end, and the sealing connector 25 is disposed at the open end 241 of the lamp tube 24a, so that the assembly efficiency of the pulse lamp 24 is high. In addition, the sealing connector 25 can seal the open end 241 of the lamp tube 24a to seal an inner space of the lamp tube 24a. On the one hand, condensed water generated in the lamp tube 24a can be prevented from affecting the light transmission of the lamp tube 24a, and on the other hand, the sealing performance and the moisture-proof performance of the lamp tube 24a can be enhanced, thereby alleviating the oxidation of the input wire harness 22. In addition, the service life of the light-emitting body 24b and the input wire harness 22 can be ensured, thus ensuring a service life of the pulse lamp 24 and a safety of the pulse lamp 24.

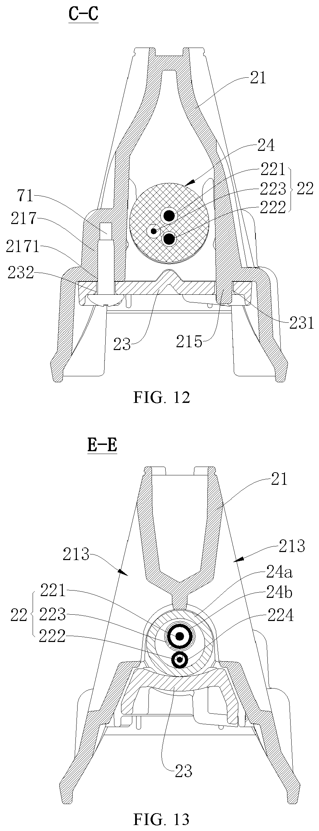

Referring to FIG. 12 to FIG. 14 and FIG. 20, according to some embodiments of the present application, the input wire harness 22 includes a positive input wire harness 221, a negative input wire harness 222, and a signal input wire harness 223. The positive input wire harness 221 and the negative input wire harness 222 are respectively connected to two opposite sides of the light-emitting body 24b. For example, the positive input wire harness 221 can be connected to a side of the light-emitting body 24b in a length direction of the light-emitting body 24b, and the negative input wire harness 222 can be connected to another side of the light-emitting body 24b in the length direction of the light-emitting body 24b. A connection between the negative input wire harness 222 and the light-emitting body 24b is located at a side of the light-emitting body 24b adjacent to the closed end 242 of the lamp tube 24a. A wire routing space 245 is defined between the lamp tube 24a and the light-emitting body 24b. The negative input wire harness 222 is routed into the sealing connector 25 through the wire routing space 245, allowing the negative input wire harness 222 to be routed in a standard manner. For example, the signal input wire harness 223 can be wound around an outer periphery of the light-emitting body 24b, and the signal input wire harness 223 can be located in the wire routing space 245. The signal input wire harness 223 can further be routed into the sealing connector 25 through the wire routing space 245. A connection between the positive input wire harness 221 and the light-emitting body 24b is located at an end of the light-emitting body 24b adj acent to the sealing connector 25. The positive input wire harness 221 is routed through the sealing connector 25.

For example, referring to FIG. 20, the light-emitting body 24b is filled with inert gas. The positive input wire harness 221 is connected to a positive electrode 243 at an end of the light-emitting body 24b, and the negative input wire harness 222 is connected to a negative electrode 244 at another end of the light-emitting body 24b. When the pulse lamp 24 is operated, a signal input wire disposed at an outer peripheral side of the light-emitting body 24b outputs a signal, to control the positive electrode 243 and the negative electrode 244 to ionize the inert gas inside the light-emitting body 24b, thereby emitting the pulsed light.

Referring to FIG. 19 and FIG. 20, according to some optional embodiments of the present application, a part of the negative input wire harness 222 located in the wire routing space 245 is a first wire harness segment 2221. At least part of the first wire harness segment 2221 facing towards the light-emitting body 24b in a radial direction of the lamp tube 24a is sleeved with an insulating sleeve 224. For example, a part of the first wire harness segment 2221 opposite to the light-emitting body 24b in the radial direction of the lamp tube 24a is sleeved with the insulating sleeve 224. Alternatively, all the first wire harness segment 2221 is sleeved with the insulating sleeve 224. The insulating sleeve 224 can separate the negative input wire harness 222 from the light-emitting body 24b, to prevent the negative input wire harness 222 from heating and oxidizing due to direct contact between the light-emitting body 24b and the negative input wire harness 222, thereby ensuring a service life of the negative input wire harness 222. The insulating sleeve 224 can further separate the negative input wire harness 222 from the signal input wire harness 223, to insulate the negative input wire harness 222 from the signal input wire harness 223, thereby preventing the negative input wire harness 222 from contacting with the signal input wire harness 223 to form a short circuit, avoiding potential safety hazards, and ensure the safety of the pulse lamp 24.

Referring to FIG. 20, in some optional embodiments of the present application, a part of the negative input wire harness 222 located in the sealing connector 25 is a second wire harness segment 2222. The insulating sleeve 224 extends into the sealing connector 25, which can ensure overall connection strength. The sealing connector 25 can limit a movement of the insulating sleeve 224, and can prevent the second wire harness segment 2222 from contacting with the signal input wire harness 223 and the positive input wire harness 221 to form a short circuit, thereby avoiding potential safety hazards.

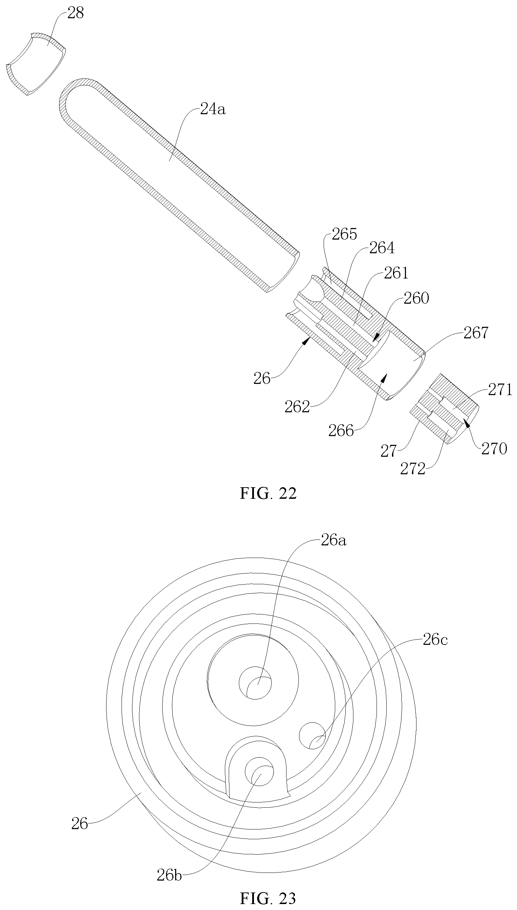

Referring to FIG. 18 to FIG. 20, according to some embodiments of the present application, the sealing connector 25 includes a sealing soft plug 26. The sealing soft plug 26 includes a blocking portion 264 received in the lamp tube 24a and blocks the open end 241 of the lamp tube 24a. The blocking portion 264 helps to seal a space in the lamp tube 24a, and prevents condensed water generated in the lamp tube 24a from affecting the light transmission of the lamp tube 24a and the service life of the light-emitting body 24b. Therefore, the oxidation of the input wire harness 22 is reduced, and the light transmission of the lamp tube 24a and the service life of the light-emitting body 24b and the input wire harness 22 can be ensured. Referring to FIG. 22, the sealing soft plug 26 further has a first routing channel 260. At least a part of the first routing channel 260 is located in the blocking portion 264. A part of the input wire harness 22 is routed along the first routing channel 260, allowing the input wire harness 22 to be routed in a standard manner. The blocking portion 264 can separate the input wire harness 22 from the lamp tube 24a, and prevent the input wire harness 22 from contacting with the lamp tube 24a, thereby preventing the lamp tube 24a from affecting the conductivity of the input wire harness 22.

Referring to FIG. 22, according to some optional embodiments of the present application, the sealing soft plug 26 has a sealing engagement groove 265 that is of an annular shape. The open end 241 of the lamp tube 24a is inserted into the sealing engagement groove 265, allowing the lamp tube 24a and the sealing soft plug 26 to tightly engage with each other, which helps to enhance the sealing performance and moisture-proof performance of the space in the lamp tube 24a. A part of the sealing soft plug 26 located at an inner peripheral side of the sealing engagement groove 265 is constructed as the blocking portion 264. The blocking portion 264 blocks the open end 241 of the lamp tube 24a.

Referring to FIG. 20, in some optional embodiments of the present application, an engagement length L between the sealing soft plug 26 and the lamp tube 24a ranges from 12 mm to 20 mm, which can ensure an engagement area between the sealing soft plug 26 and the lamp tube 24a, and ensure a pull-off force of the sealing soft plug 26. Therefore, the lamp tube 24a and the sealing soft plug 26 engage with each other tightly and are not easy to separate from each other, thereby ensuring the tightness of the space in the lamp tube 24a. For example, the engagement length L between the sealing soft plug 26 and the lamp tube 24a can be 15 mm, 16 mm, or 18 mm, and the engagement length L between the sealing soft plug 26 and the lamp tube 24a can further be a depth of the sealing engagement groove 265 in a length direction of the sealing soft plug 26.

Referring to FIG. 20 and FIG. 22, in some optional embodiments of the present application, the first routing channel 260 includes a first sub-routing channel 261, a second sub-routing channel 262, and a third sub-routing channel 263 arranged at intervals. The input wire harness 22 includes a positive input wire harness 221 routed along the first sub-routing channel 261, a negative input wire harness 222 routed along the second sub-routing channel 262, and a signal input wire harness 223 routed along the third sub-routing channel 263, allowing the positive input wire harness 221, the negative input wire harness 222, and the signal input wire harness 223 to be routed in a standard manner. By arranging the first sub-routing channel 261, the second sub-routing channel 262, and the third sub-routing channel 263 at intervals, routings of the positive input wire harness 221, the negative input wire harness 222, and the signal input wire harness 223 can be ensured to be independent of each other and not affect each other. Potential safety hazards caused by contact among the positive input wire harness 221, the negative input wire harness 222, and the signal input wire harness 223 are avoided, and the safety of the pulse lamp 24 is enhanced.

Referring to FIG. 20 and FIG. 22, in some optional embodiments of the present application, the sealing soft plug 26 has a wire outlet groove 266 formed at an axial end surface of the sealing soft plug 26 away from the light-emitting body 24b. The input wire harness 22 is led out through the wire outlet groove 266. The sealing connector 25 further includes a sealing adhesive 27 filled in the wire outlet groove 266 to at least seal a gap between the input wire harness 22 and an inner wall of the wire outlet groove 266, allowing the input wire harness 22 to insulate from the inner wall of wire outlet groove 266. Thus, the sealing adhesive 27 can further seal the interior of the lamp tube 24a, and further enhance the sealing performance and moisture-proof performance of the lamp tube 24a, thereby preventing condensed water from being generated in the lamp tube 24a, and reducing the oxidation of the input wire harness 22. An adhesive injection port 267 is defined between the input wire harness 22 and an inner peripheral wall of the wire outlet groove 266. The adhesive injection port 267 is configured for adhesive injection to form the sealing adhesive 27. In this way, an adhesive injection operation is convenient.

For example, referring to FIG. 20 and FIG. 22, according to some embodiments of the present application, the first routing channel 260 includes the first sub-routing channel 261, the second sub-routing channel 262, and the third sub-routing channel 263 arranged at intervals. A second routing channel 270 is formed in the sealing adhesive 27. The second routing channel 270 includes a fourth sub-routing channel 271, a fifth sub-routing channel 272, and a sixth sub-routing channel 273 arranged at intervals. The fourth sub-routing channel 271 is in communication with the first sub-routing channel 261. The fifth sub-routing channel 272 is in communication with the second sub-routing channel 262. The sixth sub-routing channel 273 is in communication with the third sub-routing channel 263. The input wire harness 22 includes the positive input wire harness 221 routed along the first sub-routing channel 261 and the fourth sub-routing channel 271, the negative input wire harness 222 routed along the second sub-routing channel 262 and the fifth sub-routing channel 272, and the signal input wire harness 223 routed along the third sub-routing channel 263 and the sixth sub-routing channel 273.

For example, referring to FIG. 20 and FIG. 23, a first lead hole 26a, a second lead hole 26b, and a third lead hole 26c are formed at an end of the sealing soft plug 26 close to the light-emitting body 24b. The first lead hole 26a is correspondingly in communication with the first sub-routing channel 261. The second lead hole 26b is correspondingly in communication with the second sub-routing channel 262. The third lead hole 26c is correspondingly in communication with the third sub-routing channel 263. The positive input wire harness 221 is led into the first sub-routing channel 261 through the first lead hole 26a and routed along the first sub-routing channel 261 and the fourth sub-routing channel 271. The negative input wire harness 222 is led into the second sub-routing channel 262 through the second lead hole 26b and routed along the second sub-routing channel 262 and the fifth sub-routing channel 272. The signal input wire harness 223 is led into the third sub-routing channel 263 through the third lead hole 26c and routed along the third sub-routing channel 263 and the sixth sub-routing channel 273.

Referring to FIG. 19 and FIG. 22, according to some embodiments of the present application, the pulse lamp 24 further includes a protective rubber sleeve 28 sleeved on the closed end 242 of the lamp tube 24a. The protective rubber sleeve 28 can protect the closed end 242 of the lamp tube 24a from being bumped and broken during assembly or mounting of the pulse lamp 24.

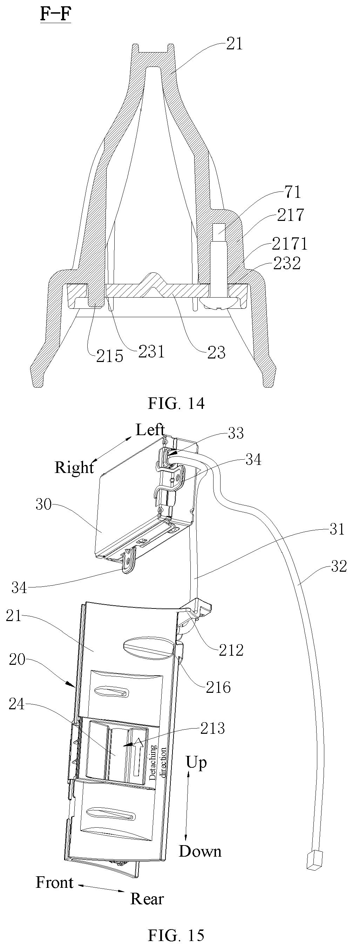

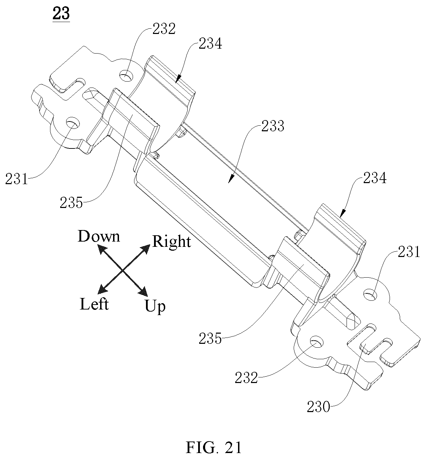

The pulse lamp 24 according to an embodiment of the present application is described below with reference to FIG. 15 to FIG. 23.

Referring to FIG. 15 to FIG. 23, in this embodiment, the pulse lamp 24 includes the lamp tube 24a, the light-emitting body 24b, the protective rubber sleeve 28, and the sealing connector 25. The lamp tube 24a has the open end and the closed end. The protective rubber sleeve 28 is sleeved on the closed end 242 of the lamp tube 24a. The light-emitting body 24b is disposed in the lamp tube 24a. Two ends of the light-emitting body 24b in the length direction of the light-emitting body 24b are connected with the input wire harness 22. The sealing connector 25 is disposed at the open end 241 of the lamp tube 24a. The input wire harness 22 is led out through the sealing connector 25.

The input wire harness 22 includes the positive input wire harness 221, the negative input wire harness 222, and the signal input wire harness 223. The positive input wire harness 221 can be connected to a side of the light-emitting body 24b in the length direction of the light-emitting body 24b. The connection between the positive input wire harness 221 and the light-emitting body 24b is located at a side of the light-emitting body 24b adjacent to the open end 241 of the lamp tube 24a. The positive input wire harness 221 is connected to the positive electrode 243 at an end of the light-emitting body 24b. The negative input wire harness 222 can be connected to another side of the light-emitting body 24b in the length of the light-emitting body 24b. The connection between the negative input wire harness 222 and the light-emitting body 24b is located at the side of the light-emitting body 24b adjacent to the closed end 242 of the lamp tube 24a. The negative input wire harness 222 connected to the negative electrode 244 at another end of the light-emitting body 24b. The wire routing space 245 is defined between the lamp tube 24a and the light-emitting body 24b. The signal input wire harness 223 is wound around the outer periphery of the light-emitting body 24b. The signal input wire harness 223 is located in the wire routing space 245. Both the signal input wire harness 223 and the negative input wire harness 222 are routed into the sealing connector 25 through the wire routing space 245.

The negative input wire harness 222 includes the first wire harness segment 2221 and the second wire harness segment 2222, the part of the negative input wire harness 222 located in the wire routing space 245 is the first wire harness segment 2221. The part of the first wire harness segment 2221 facing towards the light-emitting body 24b in the radial direction of the lamp tube 24a is sleeved with the insulating sleeve 224. The part of the negative input wire harness 222 located in the sealing connector 25 is the second wire harness segment 2222. The insulating sleeve 224 extends into the sealing connector 25, and is sleeved on an outer peripheral side of the second wire harness segment 2222.

The sealing connector 25 includes the sealing soft plug 26 and the sealing adhesive 27. The sealing soft plug 26 includes the sealing engagement groove 265, the first routing channel 260, and the blocking portion 264. The sealing engagement groove 265 is of an annular shape. The open end 241 of the lamp tube 24a is inserted into the sealing engagement groove 265. The depth of the sealing engagement groove 265 in the length direction of the sealing soft plug 26 is 16 mm, and the engagement length L between the sealing soft plug 26 and the lamp tube 24a is further 16 mm. The part of the sealing soft plug 26 located at the inner peripheral side of the sealing engagement groove 265 is constructed as the blocking portion 264. The blocking portion 264 is received in the lamp tube 24a and blocks the open end 241 of the lamp tube 24a. The sealing soft plug 26 further has the first routing channel 260. At least a part of the first routing channel 260 is located in the blocking portion 264. A part of the input wire harness 22 is routed along the first routing channel 260. The first routing channel 260 includes the first sub-routing channel 261, the second sub-routing channel 262, and the third sub-routing channel 263 arranged at intervals. The positive input wire harness 221 is routed along the first sub-routing channel 261. The negative input wire harness 222 is routed along the second sub-routing channel 262. The signal input wire harness 223 is routed along the third sub-routing channel 263. The first lead hole 26a, the second lead hole 26b, and the third lead hole 26c are formed at the end of the sealing soft plug 26 close to the light-emitting body 24b. The first lead hole 26a is correspondingly in communication with the first sub-routing channel 261, the second lead hole 26b is correspondingly in communication with the second sub-routing channel 262, and the third lead hole 26c is correspondingly in communication with the third sub-routing channel 263.

The wire outlet groove 266 is formed at the axial end surface of the sealing soft plug 26 away from the light-emitting body 24b. The input wire harness 22 is led out through the wire outlet groove 266. The adhesive injection port 267 is defined between the input wire harness 22 and the inner peripheral wall of the wire outlet groove 266. The adhesive injection port 267 is configured for adhesive injection to form the sealing adhesive 27. The sealing adhesive 27 is filled in the wire outlet groove 266 to at least seal the gap between the input wire harness 22 and the inner wall of the wire outlet groove 266. The second routing channel 270 is formed in the sealing adhesive 27. The second routing channel 270 includes the fourth sub-routing channel 271, the fifth sub-routing channel 272, and the sixth sub-routing channel 273 arranged at intervals. The fourth sub-routing channel 271 is in communication with the first sub-routing channel 261. The fifth sub-routing channel 272 is in communication with the second sub-routing channel 262. The sixth sub-routing channel 273 is in communication with the third sub-routing channel 263. The positive input wire harness 221 is led into the first sub-routing channel 261 through the first lead hole 26a and routed along the first sub-routing channel 261 and the fourth sub-routing channel 271. The negative input wire harness 222 is led into the second sub-routing channel 262 through the second lead hole 26b and routed along the second sub-routing channel 262 and the fifth sub-routing channel 272. The signal input wire harness 223 is led into the third sub-routing channel 263 through the third lead hole 26c and routed along the third sub-routing channel 263 and the sixth sub-routing channel 273.

When assembling the pulse lamp 24, the insulating sleeve 224 can be sleeved on the outer peripheral side of the negative input wire harness 222 first, and then the light-emitting body 24b and the input wire harness 22 as a whole are inserted into the lamp tube 24a from the open end 241 of the lamp tube 24a. The input wire harness 22 is led out from the open end 241 of the lamp tube 24a. Next, the sealing soft plug 26 is disposed at the open end 241 of the lamp tube 24a, and the open end 241 of the lamp tube 24a is inserted into the sealing engagement groove 265 of the sealing soft plug 26. The input wire harness 22 is routed through the first routing channel 260 of the sealing soft plug 26. Finally, the adhesive is injected into the wire outlet groove 266 of the sealing soft plug 26 through the adhesive injection port 267. After the adhesive is solidified, the sealing adhesive 27 can be formed. The sealing adhesive 27 is filled in the wire outlet groove 266 to at least seal the gap between the input wire harness 22 and the inner wall of the wire outlet groove 266, thereby completing the assembly of the pulse lamp 24.

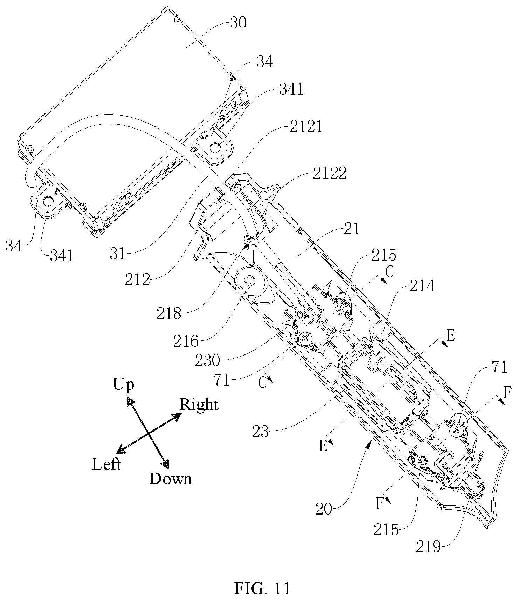

Referring to FIG. 11 to FIG. 21, a pulse sterilization module 20 according to the embodiments in a second aspect of the present application includes a lamp cover 21, a lamp support 23, and the pulse lamp 24 according to the embodiments in the first aspect of the present application. The pulse lamp 24 is disposed in the lamp cover 21, allowing an overall structure to be compact. The pulse lamp 24 is fixedly mounted in the lamp cover 21 through the lamp support 23, allowing mounting stability of the pulse lamp 24 to be strong. The lamp cover 21 can further support and protect the pulse lamp 24.

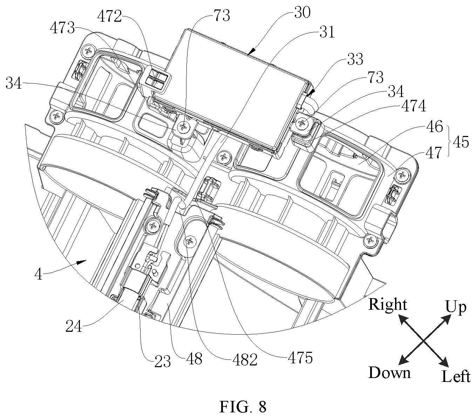

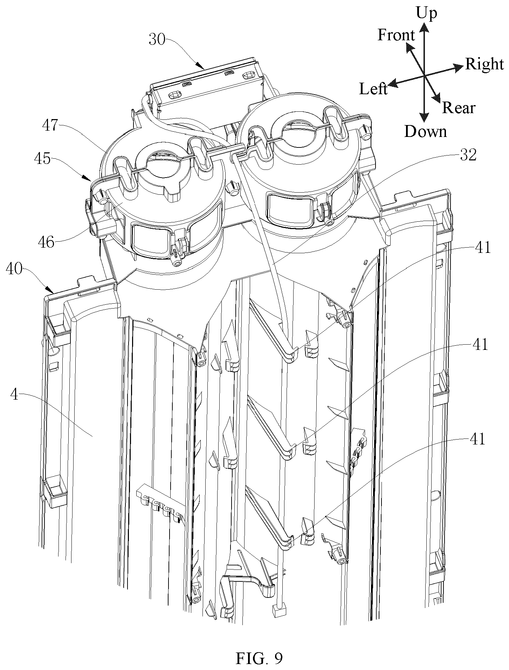

For example, referring to FIG. 8 to FIG. 11, the lamp cover 21 is provided with two positioning posts 215 and two fixing posts 217 at an inner wall of the lamp cover 21. The two positioning posts 215 are arranged diagonally, and the two fixing posts 217 are arranged diagonally. Each fixing post 217 has one first fixing hole 2171. Two first positioning holes 231 and two second fixing holes 232 are formed at the lamp support 23. The two first positioning holes 231 correspond to the two positioning posts 215 one by one. The two second fixing holes 232 correspond to the two first fixing holes 2171 one by one. The lamp support 23 can be positioned in the lamp cover 21 by inserting the positioning posts 215 into the first positioning holes 231. The lamp support 23 can be fixedly mounted to the lamp cover 21 by a first fastener 71 passing through the second fixing hole 232 and the first fixing hole 2171. Since the pulse lamp 24 is disposed at the lamp support 23, the pulse lamp 24 can be fixedly mounted to the lamp cover 21. The pulse lamp 24 can be located in a space defined by the lamp support 23 and the lamp cover 21. A light outlet 213 is formed at the lamp cover 21. The pulsed light emitted by the pulse lamp 24 can be irradiated through the light outlet 213, and the light outlet 213 on the lamp cover 21 can limit a irradiation range of the pulsed light .

According to the pulse sterilization module 20 of the embodiments of the present application, since the lamp tube 24a has the open end and the closed end, and the sealing connector 25 is disposed at the open end 241 of the lamp tube 24a, so that the assembly efficiency of the pulse lamp 24 is high. In addition, the sealing connector 25 can seal the open end 241 of the lamp tube 24a to seal the inner space of the lamp tube 24a. On the one hand, condensed water generated in the lamp tube 24a can be prevented from affecting the light transmission of the lamp tube 24a, and on the other hand, the sealing performance and the moisture-proof performance of the lamp tube 24a can be enhanced, thereby alleviating the oxidation of the input wire harness 22. In addition, the service life of the light-emitting body 24b and the input wire harness 22 can be ensured, thus ensuring the service life of the pulse lamp 24 and the safety of the pulse lamp 24.

Referring to FIG. 11 and FIG. 18 to FIG. 21, according to some embodiments of the present application, the lamp support 23 is provided with a wire routing buckle 230 at an end of the lamp support 23 adj acent to the sealing connector 25. A part of the input wire harness 22 located outside the pulse lamp 24 is routed through the wire routing buckle 230, allowing the input wire harness 22 to be routed in a standard manner and a routing structure to be simple.

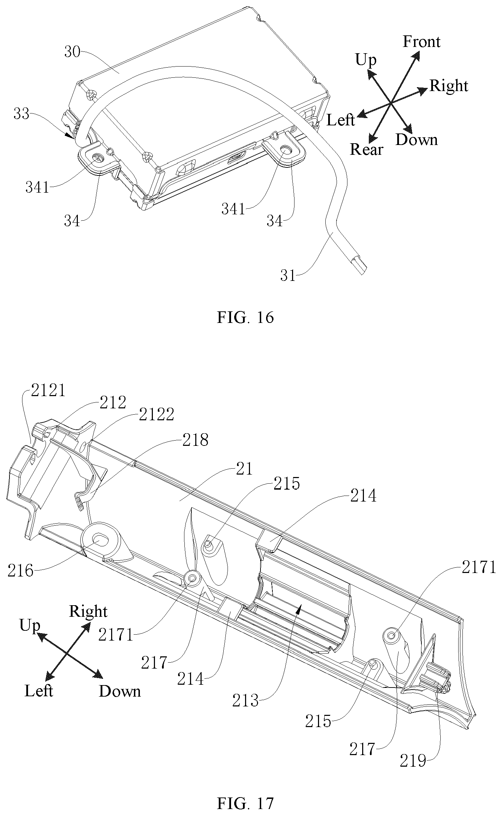

For example, referring to FIG. 11 and FIG. 17, according to a specific embodiment of the present application, a top of the lamp cover 21 has a support portion 212. The support portion 212 has one first wire routing groove 2121 formed at an outer edge of the support portion 212, and is provided with one third wire routing buckle 218 at a lower side of the support portion 212. The input wire harness 22 that is routed through the wire routing buckle 230 is further routed through the third wire routing buckle 218, and is led out the pulse sterilization module 20 from the first wire routing groove 2121. Referring to FIG. 18 and FIG. 19, an input wire protective sleeve 225 is sleeved outside the input wire harness 22. The input wire protective sleeve 225 can prevent the input wire harness 22 from being scratched, to facilitate clamping wiring. The input wire harness 22 and the input wire protective sleeve 225 are constructed as a first connection wire harness 31.

Referring to FIG. 18 to FIG. 21, according to some embodiments of the present application, the lamp support 23 has a lamp body groove 233 and is provided with positioning clips 234. The positioning clips 234 are located at two opposite sides of the lamp body groove 233 in a length direction of the lamp body groove 233. Two ends of the lamp tube 24a are positioned by the positioning clips 234. In this way, a positioning structure is relatively simple, and the stability is relatively strong. For example, the lamp support 23 is provided with one lamp body groove 233 and two positioning clips 234, and the lamp body groove 233 is positioned between the two positioning clips 234. Each positioning clip 234 includes positioning clip plates 235 arranged oppositely and spaced apart from each other in a width direction of the lamp body groove 233.

For example, referring to FIG. 18 and FIG. 19, according to a specific embodiment of the present application, the pulse lamp 24 further includes the protective rubber sleeve 28. The protective rubber sleeve 28 is sleeved on the closed end 242 of the lamp tube 24a. The sealing connector 25 is disposed at the open end 241 of the lamp tube 24a. The sealing connector 25 and the protective rubber sleeve 28 can engage with the positioning clips 234. The protective rubber sleeve 28 and the sealing connector 25 can be positioned on the lamp support 23 through positioning clips 234, allowing the two ends of the lamp tube 24a to be positioned on the lamp support 23. The positioning clips 234 can clamp the two ends of the pulse lamp 24, allowing the pulse lamp 24 to be mounted at the lamp support 23. The mounting stability is strong, and the mounting structure is simpler.

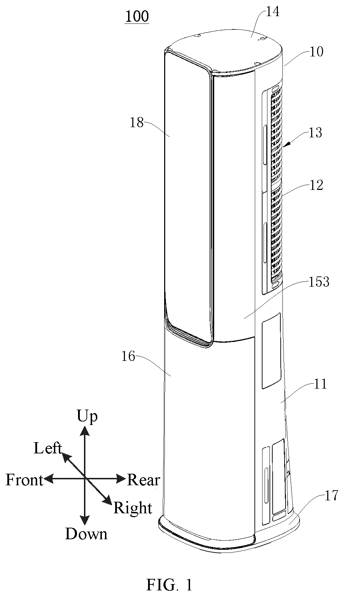

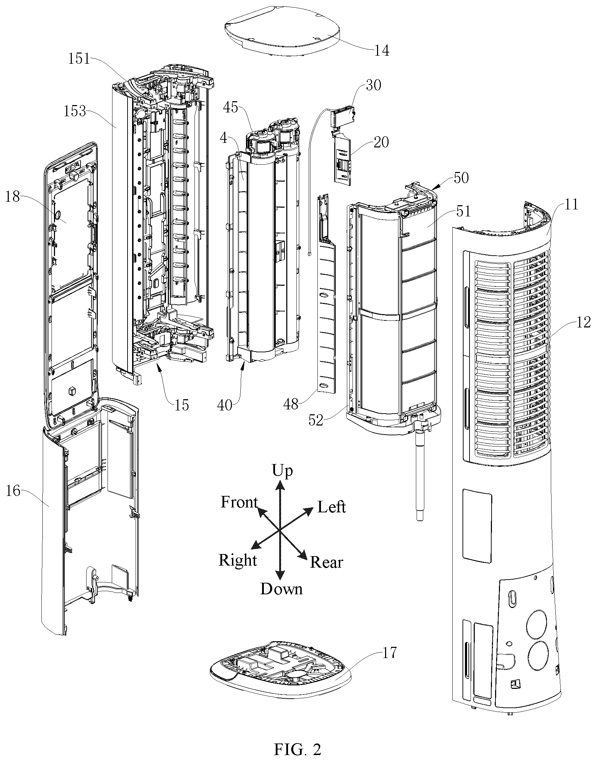

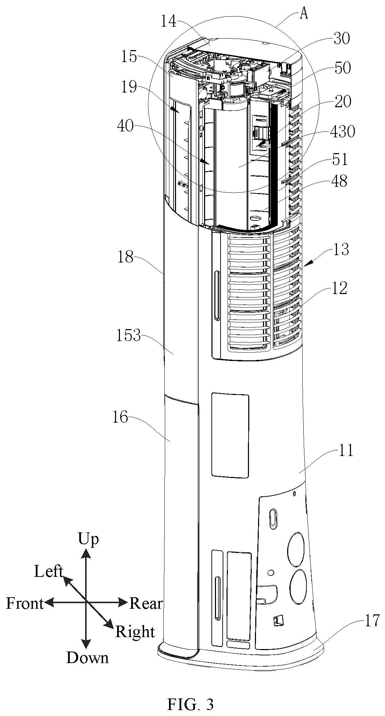

Referring to FIG. 1 to FIG. 6, an air conditioner according to embodiments in a third aspect of the present application includes a housing 10, a heat exchanger component 50, an air duct component 40, and the pulse sterilization module 20 according to the embodiments in the first aspect of the present application. The housing 10 has an air inlet 13 and an air outlet 19. The air inlet 13 and the air outlet 19 are respectively in communication with the room for facilitating airflow circulation. The heat exchanger component 50 and the air duct component 40 are arranged in the housing 10. The pulse sterilization module 20 is disposed in the housing 10 and adapted to emit pulsed light into the housing 10. When the pulsed light is irradiated on the surface of microorganisms (such as bacteria and viruses) in the housing 10, substances in the microorganisms can be changed and inactivated, allowing various microorganisms in the housing 10 to be completely killed, thereby realizing purification and sterilization of various parts and air in the housing 10. Therefore, a sterilization effect is good and a sterilization efficiency is high. In addition, some harmful gases (such as VOC gases) in the air can further be decomposed under the irradiation of the pulsed light, which can purify the air and improve the quality of the air blown into the room.

For example, referring to FIG. 1 to FIG. 3, the air conditioner can be a split-type air conditioner, for example, the air conditioner can be a split-floor-type air conditioner. The air conditioner includes an indoor unit 100 and an outdoor unit. The indoor unit 100 of the air conditioner includes the above-described housing 10, the heat exchanger component 50, the air duct component 40, and the pulse sterilization module 20. The indoor unit 100 of the air conditioner can be a cabinet unit. The housing 10 can include a rear housing 11, a top cover 14, an air outlet frame 15, an upper panel 18, a lower panel 16, and a base 17. The upper panel 18 is located at a front side of the air outlet frame 15. The upper panel 18 and the air outlet frame 15 are located at an upper side of the lower panel 16. The upper panel 18, the air outlet frame 15, and the lower panel 16 are located at a front side of the rear housing 11. The top cover 14 is covered on a top of the air outlet frame 15 and a top of the rear housing 11. The base 17 is located at a bottom of the lower panel 16 and a bottom of rear housing 11. The air outlet frame 15 can be detachably connected to the rear housing 11. The lower panel 16 can be detachably connected to the rear housing 11, and the top cover 14 can further be detachably connected to the air outlet frame 15 and the rear housing 11.

For example, referring to FIG. 1 to FIG. 6, the air inlet 13 can be formed at the rear housing 11. The air inlet 13 is in communication with the room. Air inlet grilles 12 are arranged at the air inlet 13. The heat exchanger component 50 can be located at a front side of the air inlet 13. The air duct component 40 can be located at a front side of the heat exchanger component 50. The air outlet frame 15 can be located at a front side of the air duct component 40. The heat exchanger component 50 can include a heat exchanger 51, a heat exchanger support 51, and an electric auxiliary heating component 53. The heat exchanger 51 and the electric auxiliary heating component 53 are both arranged at the heat exchanger support 51. The electric auxiliary heating component 53 can extend in an up-down direction. The air outlet frame 15 can include a frame 151 and air guide plates 153. The air guide plates 153 are located at left and right sides of the frame 151, and are rotatably connected to the upper panel 18 to open and close the air outlets 19 located at left and right sides of the upper panel 18. A plurality of louver blades 152 is arranged at the air outlet 19, and is rotatably arranged in the air outlet frame 15. The louver blades 152 can change a blowing direction of air at the air outlet 19. When the air conditioner is in operation, air is drawn into the housing 10 from the air inlet 13 and passes through the heat exchanger component 50 for heat exchange, and the heat exchanged air is blown into the room through the air outlet 19 under a drive of the air duct component 40 to cool down or warm up the room.

According to the air conditioner of the embodiments of the present application, by providing the above-described pulse sterilization module 20 in the air conditioner, since the lamp tube 24a has the open end and the closed end, and the sealing connector 25 is disposed at the open end 241 of the lamp tube 24a, so that the assembly efficiency of the pulse lamp 24 is high. In addition, the sealing connector 25 can seal the open end 241 of the lamp tube 24a to seal the inner space of the lamp tube 24a. On the one hand, condensed water generated in the lamp tube 24a can be prevented from affecting the light transmission of the lamp tube 24a, and on the other hand, the sealing performance and the moisture-proof performance of the lamp tube 24a can be enhanced, thereby alleviating the oxidation of the input wire harness 22. In addition, the service life of the light-emitting body 24b and the input wire harness 22 can be ensured, thus ensuring the service life of the pulse lamp 24 and the safety of the pulse lamp 24.

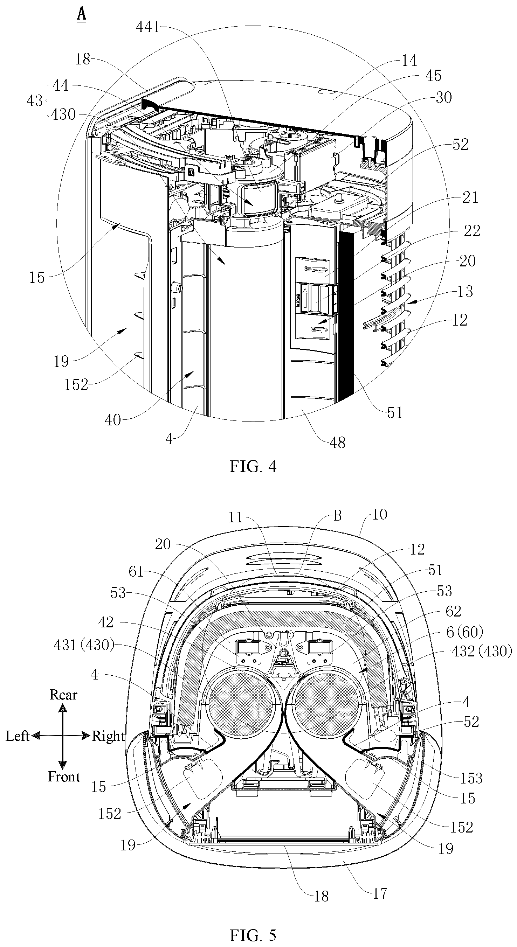

Referring to FIG. 5 to FIG. 10, according to some embodiments of the present application, an air duct cavity 60 is defined between the heat exchanger component 50 and the air duct component 40. The air duct component 40 includes a fan 43, an air duct volute 4, and an air duct partition 48. The air duct partition 48 is connected to a side of the air duct volute 4 adjacent to the heat exchanger component 50 and abuts against the heat exchanger component 50 to divide the air duct cavity 60 into two air duct sub-cavities 6. In this way, airflows of the two air duct sub-cavities 6 can be prevented from running around, and the uniformity of left and right wind speeds can be ensured. The fan 43 has impellers 430 disposed in the air duct sub-cavities 6, allowing an overall structure to be compact and a space in housing 10 to be saved.

Referring to FIG. 4, FIG. 7, and FIG. 8, the pulse sterilization module 20 is disposed at an upper part of the air duct partition 48. The pulsed light emitted from the pulse lamp 24 in the pulse sterilization module 20 can be irradiated on upper parts of the air duct component 40, the heat exchanger component 50, and the air duct sub-cavities 6 to purify and sterilize the upper parts of the air duct component 40 and the heat exchanger component 50 and further purify and sterilize air passing through the air duct sub-cavities 6. For example, the pulse sterilization module 20 can be fixedly mounted to the upper part of the air duct partition 48 by fixedly mounting the lamp cover 21 to the upper part of the air duct partition 48.

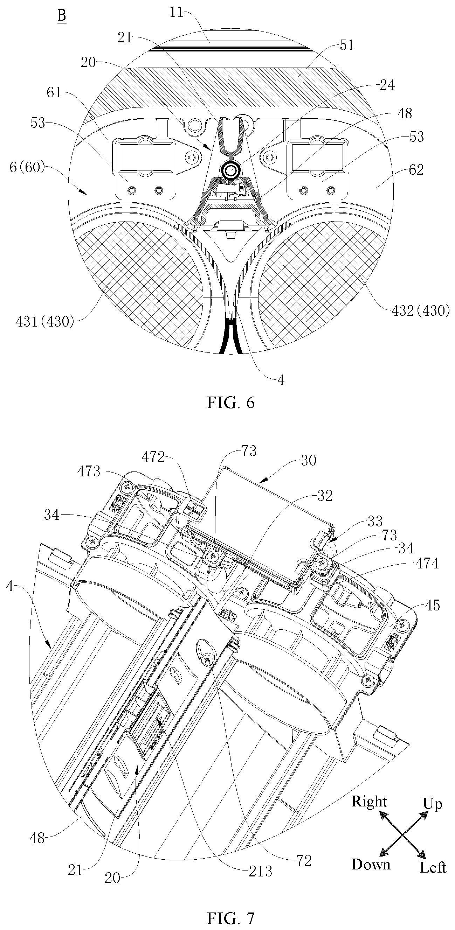

For example, referring to FIG. 7, FIG. 10, and FIG. 17, according to some specific embodiments of the present application, a mounting notch 480 is formed at the upper part of the air duct partition 48. At least a part of the pulse sterilization module 20 is received in the mounting notch 480. A wall of the mounting notch 480 is constructed as a mounting wall 481. One positioning protrusion 483 is disposed at a top surface of the mounting wall 481. Two first clip grooves 484 are formed at a surface of the mounting wall 481 facing the heat exchanger component 50. The mounting wall 481 has one first limiting groove 485 formed at a bottom surface of the mounting wall 481. The top of the lamp cover 21 has a support portion 212, and the support portion 212 has one second positioning hole 2122. The lamp cover 21 is provided with two first clips 214 at an inner side wall of the lamp cover 21, and the two first clips 214 are spaced apart from each other in a left-right direction. The lamp cover 21 is provided with one first limiting protrusion 219 at a bottom surface of the lamp cover 21. The positioning protrusion 483 corresponds to the second positioning hole 2122. The first clip groove 484 corresponds to the first clip 214. The first limiting groove 485 corresponds to the first limiting protrusion 219. By engaging the positioning protrusion 483 with the second positioning hole 2122, the first clip groove 484 with the first clip 214, and the first limiting groove 485 with the first limiting protrusion 219, the pulse sterilization module 20 can be clipped onto the mounting wall 481. The lamp cover 21 further has one third fixing hole 216 formed at the inner wall of the lamp cover 21, and the air duct partition 48 has one fourth fixing hole 486 formed at the upper part of the air duct partition 48. The fourth fixing hole 486 corresponds to the third fixing hole 216. A second fastener 72 is adapted to pass through the third fixing hole 216 and the fourth fixing hole 486. Therefore, the lamp cover 21 can be fixedly mounted to the upper part of the air duct partition 48, thereby fixedly mounting the pulse sterilization module 20 to the upper part of the air duct partition 48.

For example, referring to FIG. 5 and FIG. 6, according to some specific embodiments of the present application, the air duct partition 48 is connected at a rear side of the air duct volute 4. The heat exchanger component 50 is located at a rear side of the air duct partition 48. The air duct partition 48 abuts against the heat exchanger component 50 in a front-rear direction to divide the air duct cavity 60 into a first air duct sub-cavity 61 and a second air duct sub-cavity 62. The first air duct sub-cavity 61 is located at a left side of the second air duct sub-cavity 62. The fan 43 includes two impellers 430 and two motors 44. The two impellers 430 are respectively a first impeller 431 and a second impeller 432. The first impeller 431 is disposed in the first air duct sub-cavity 61, and the second impeller 432 is disposed in the second air duct sub-cavity 62. The two motors 44 are respectively a first motor 441 and a second motor 442. The first motor 441 is disposed at an upper side of the first impeller 431 and connected to the first impeller 431 to drive the first impeller 431 to rotate. The second motor 442 is disposed at an upper side of the second impeller 432 and connected to the second impeller 432 to drive the second impeller 432 to rotate.

Referring to FIG. 8, FIG. 11, and FIG. 15, according to some optional embodiments of the present application, the air conditioner further includes a pulse electric control box 30 electrically connected to the pulse sterilization module 20. The pulse electric control box 30 can output a signal and energy to control an operation of the pulse lamp 24, and can further control an operation time of the pulse sterilization module 20 and a frequency of the pulsed light emitted by the pulse sterilization module 20. For example, the pulse electric control box 30 includes a first connection wire harness 31 and a second connection wire harness 32, both of which are led out from a wire outlet hole 33 of the pulse electric control box 30. The first connection wire harness 31 can include the positive input wire harness 221, the negative input wire harness 222, and the signal wire harness. The pulse electric control box 30 is electrically connected to the pulse lamp 24 of the pulse sterilization module 20 through the first connection wire harness 31, and can transmit energy and electrical signals for the pulse lamp 24 to emit the pulsed light.

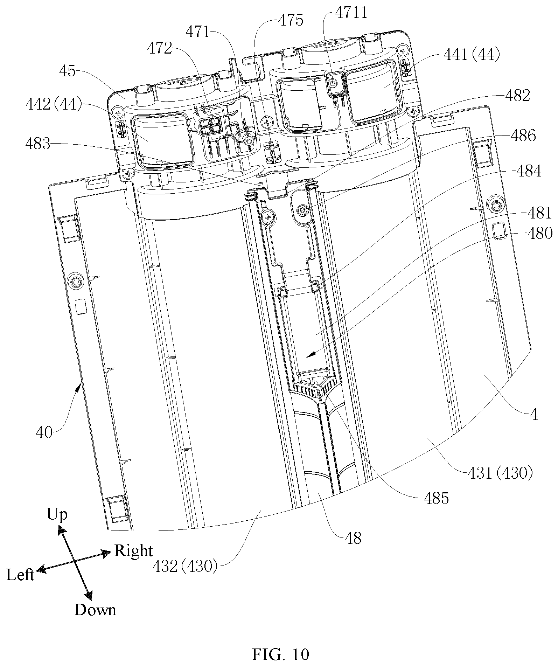

Referring to FIG. 8 and FIG. 10, the air duct volute 4 is provided with a motor cover 45 at an outer wall of the air duct volute 4. The fan 43 has a motor 44 disposed in the motor cover 45 and connected to the impeller 430. For example, the motor cover 45 includes a front cover body 46 and a rear cover body 47 connected in front and rear. The rear cover body 47a has one second wire routing groove 475 at an outer wall of the rear cover body 47. The air duct partition 48 is located at a lower side of the motor cover 45, and the air duct partition 48 has one third wire routing groove 482 formed at a top surface of the air duct partition 48. After the first connection wire harness 31 is led out from the wire outlet hole 33 of the pulse electric control box 30, the first connection wire harness 31 is routed along the second wire routing groove 475 and the third wire routing groove 482, passes through the first wire routing groove 2121 on the lamp cover 21, and is electrically connected to the pulse sterilization module 20. A plurality of second wire routing buckles 41 arranged at intervals in the up-down direction is arranged at a front side wall of the air duct volute 4. After the second connection wire harness 32 is led out from the wire outlet hole 33 of the pulse electric control box 30, the second connection wire harness 32 routes around the motor cover 45 and to the front side of the air duct volute 4, and is routed along the second wire routing buckle 41.

Referring to FIG. 7 and FIG. 8, the pulse electric control box 30 is fixedly mounted to the motor cover 45. For example, referring to FIG. 16, the pulse electric control box 30 is provided with two mounting lugs 34, and each mounting lug 34 has one first mounting hole 341. Referring to FIG. 7, FIG. 8, and FIG. 10, the rear cover body 47 is provided with a limiting buckle 472, a first limiting rib plate 473, and a second limiting rib plate 474 at an outer wall of the rear cover body 47. The limiting buckle 472 abuts against a surface of the pulse electric control box 30 facing away from the motor cover 45. The first limiting rib plate 473 and the second limiting rib plate 474 are both located at an outer peripheral side of the pulse electric control box 30. The limiting buckle 472, the first limiting rib plate 473, and the second limiting rib plate 474 collectively limit the pulse electric control box 30. The outer wall of the rear cover body 47 is provided with two first mounting posts 471. Each first mounting post 471 has one second mounting hole 4711. The second mounting hole 4711 corresponds to the first mounting hole 341. A third fastener 73 passes through the first mounting hole341 and the second mounting hole 4711 to fixedly mount the pulse electric control box 30 to the motor cover 45.

In the description of the present application, it should be understood that the orientation or position relationship indicated by the terms such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", and "circumferential" should be construed to refer to the orientation or the position as shown in the drawings, and is only for the convenience of describing the present application and simplifying the description, rather than indicating or implying that the pointed apparatus or element must have a specific orientation, or be constructed and operated in a specific orientation, and therefore cannot be understood as a limitation of the present application.

In the description of the present application, "plurality" means two or more.

In the description of the present application, the first feature "above" the second feature means that the first feature is directly above and obliquely above the second feature, or simply means that the level of the first feature is higher than that of the second feature.

In the description of this specification, description with reference to the terms "an embodiment", "some embodiments", "exemplary embodiments", "examples" "specific examples", or "some examples" etc., mean that specific features, structure, materials or characteristics described in conjunction with the embodiment or example are included in at least one embodiment or example of the present application. In this specification, the schematic representations of the above terms do not necessarily refer to the same embodiment or example. Moreover, the described specific features, structures, materials or characteristics may be combined in any one or more embodiments or examples in a suitable manner.

Although embodiments of the present application have been illustrated and described, it is conceivable for those of ordinary skill in the art that various changes, modifications, replacements, and variations can be made to these embodiments without departing from the principles and spirit of the present application. The scope of the present application shall be defined by the claims as appended and their equivalents.

## Claims

1. A pulse lamp, comprising:
a lamp tube having an open end and a closed end;
a light-emitting body disposed in the lamp tube, and connected with an input wire harness; and
a sealing connector disposed at the open end of the lamp tube, the input wire harness being led out through the sealing connector.

2. The pulse lamp according to claim 1, wherein:
the input wire harness comprises a positive input wire harness, a negative input wire harness, and a signal input wire harness;
the positive input wire harness and the negative input wire harness are respectively connected to two opposite sides of the light-emitting body;
a connection between the negative input wire harness and the light-emitting body is located at a side of the light-emitting body adjacent to the closed end of the lamp tube; and
a wire routing space is defined between the lamp tube and the light-emitting body, the negative input wire harness being routed into the sealing connector through the wire routing space.

3. The pulse lamp according to claim 2, wherein a part of the negative input wire harness located in the wire routing space is a first wire harness segment, and at least part of the first wire harness segment facing towards the light-emitting body in a radial direction of the lamp tube is sleeved with an insulating sleeve.

4. The pulse lamp according to claim 3, wherein a part of the negative input wire harness located in the sealing connector is a second wire harness segment, the insulating sleeve extending into the sealing connector and being sleeved on an outer peripheral side of the second wire harness segment.

5. The pulse lamp according to any one of claims 1 to 4, wherein:
the sealing connector comprises a sealing soft plug;
the sealing soft plug comprises a blocking portion received in the lamp tube and blocking the open end of the lamp tube; and
the sealing soft plug further has a first routing channel, at least part of the first routing channel being located in the blocking portion, and a part of the input wire harness being routed along the first routing channel.

6. The pulse lamp according to claim 5, wherein:
the sealing soft plug has a sealing engagement groove that is of an annular shape, the open end of the lamp tube being inserted into the sealing engagement groove; and
a part of the sealing soft plug located at an inner peripheral side of the sealing engagement groove is constructed as the blocking portion.

7. The pulse lamp according to claim 5, wherein an engagement length between the sealing soft plug and the lamp tube ranges from 12 mm to 20 mm.

8. The pulse lamp according to claim 5, wherein:
the first routing channel comprises a first sub-routing channel, a second sub-routing channel, and a third sub-routing channel arranged at intervals; and
the input wire harness comprises a positive input wire harness routed along the first sub-routing channel, a negative input wire harness routed along the second sub-routing channel, and a signal input wire harness routed along the third sub-routing channel.

9. The pulse lamp according to claim 5, wherein:
the sealing soft plug has a wire outlet groove formed at an axial end surface of the sealing soft plug away from the light-emitting body, the input wire harness being led out through the wire outlet groove;
the sealing connector further comprises a sealing adhesive filled in the wire outlet groove to at least seal a gap between the input wire harness and an inner wall of the wire outlet groove; and
an adhesive injection port is defined between the input wire harness and an inner peripheral wall of the wire outlet groove, the adhesive injection port being configured for adhesive injection to form the sealing adhesive.

10. The pulse lamp according to any one of claims 1 to 9, further comprising a protective rubber sleeve sleeved on the closed end of the lamp tube.

11. A pulse sterilization module, comprising:
a lamp cover;
the pulse lamp according to any one of claims 1 to 10, disposed in the lamp cover; and
a lamp support, the pulse lamp being fixedly mounted in the lamp cover through the lamp support.

12. The pulse sterilization module according to claim 11, wherein the lamp support is provided with a wire routing buckle at an end of the lamp support adj acent to the sealing connector, a part of the input wire harness located outside the pulse lamp being routed through the wire routing buckle.

13. The pulse sterilization module according to claim 11 or 12, wherein the lamp support has a lamp body groove and is provided with positioning clips, the positioning clips being located at two opposite sides of the lamp body groove in a length direction of the lamp body groove, and two ends of the lamp tube being positioned by the positioning clips.

14. An air conditioner, comprising:
a housing having an air inlet and an air outlet;
a heat exchanger component and an air duct component that are arranged in the housing;
the pulse sterilization module according to any one of claims 11 to 13, disposed in the housing and adapted to emit pulsed light into the housing.

15. The air conditioner according to claim 14, wherein:
an air duct cavity is defined between the heat exchanger component and the air duct component; and
the air duct component comprises a fan, an air duct volute, and an air duct partition, wherein:
the air duct partition is connected to a side of the air duct volute adjacent to the heat exchanger component and abuts against the heat exchanger component to divide the air duct cavity into two air duct sub-cavities;
the fan has an impeller disposed in the two air duct sub-cavities; and
the pulse sterilization module is disposed at an upper part of the air duct partition.

16. The air conditioner according to claim 15, further comprising a pulse electric control box electrically connected to the pulse sterilization module, wherein:
the air duct volute is provided with a motor cover at an outer wall of the air duct volute, the pulse electric control box being fixedly mounted at the motor cover; and
the fan has a motor disposed in the motor cover and connected to the impeller.
